# EUROPEAN PATENT APPLICATION

(11) **EP 3 195 915 A1**
(43) Date of publication of application: **26.07.2017**
(21) Application number: 16152117.4
(22) Date of filing: 20.01.2016
(51) Int. Cl.: B01D 3/00, B01D 3/14, B01D 53/22, B01D 61/36, C07C 29/76, C07C 29/80, C07C 31/08

(54) **A SYSTEM FOR THE PURIFICATION OF AN ORGANIC SOLVENT AND A PROCESS FOR THE USE THEREOF**

(71) Applicant: Sulzer Chemtech AG, 8404 Winterthur (CH)
(72) Inventor: Raiser, Thomas, 68680 Kembs (FR)
(74) Representative: Manitz Finsterwald Patentanwälte PartmbB

(57) **Abstract**

A system 1 for the purification of an organic solvent, preferably an alcohol, comprising a first distillation column 10, a second distillation column 20, a vapor permeation unit 30 suitable for the dehydration of an organic solvent, wherein the system 1 further comprises a first heat integration sub-system 100 for exploiting both a sensible heat and optionally a latent heat, a second and a third heat integration sub-systems 200 and 300 for exploiting a latent heat, and wherein there is either a parallel configuration of the system 1 with a split feeding into both the first and the second distillation columns 10 and 20, or there is a series configuration of the system 1 in which there is feeding into only the first distillation column 10.

## Description

### BACKGROUND OF THE INVENTION

The present invention relates to a system for the purification of an organic solvent. The present invention also relates to a process for using said system.

The production of dry solvents from raw mixtures containing water is often costly and complex in terms of the necessary equipment and processing. The preparation of dry ethanol is a good example of industrial and economic importance. In the conventional process, the raw fermentation broth (or alternatively the product of an industrial chemical synthesis) is stripped under moderate vacuum in a beer still. Overhead vapor from the beer still is then sent to a rectification column that produces an overhead product close to the azeotropic composition (about 93 wt % ethanol) and a bottoms product, which is essentially water. The condensed product from the top of the rectification column is subsequently evaporated under pressure and fed to a molecular sieve dryer, which produces ethanol of about 99 wt % or higher purity. Such processes consume already about 100 million Btu/h to produce 50 million gallons per year of purified ethanol from a feed containing about 11 wt % ethanol. If the concentration of ethanol in the feed is lower, for example from about 4 to 5%, the energy consumption of the processes can rise quite significantly, often exceeding the energy content of the dry ethanol produced.

A system for producing ethanol from an organic source and that operates to purify and dry ethanol from a beer source is known from US2007/0000769 A1. The system for producing substantially anhydrous ethanol consists of a series configuration of a first distillation stripping column followed by a second distillation rectifying column and finally a molecular sieve dryer. As described above such systems have the disadvantage of having very high energy consumptions, as well as having large recycle stream volumes. Furthermore the use of molecular sieve dryers means that the dehydration process can only be operated semi-continuously as the molecular sieves require regeneration. Thus typically at least two molecular sieve beds are required with one typically being charged while the other one is being regenerated.

The production of dry solvents from raw aqueous mixtures based on a combination of distillation followed by treatment of the overhead vapor by membrane separation is known also in the art, for example, as disclosed in US 9,138,678 B2, which discloses a process including distillation in two columns connected in series and operated at sequentially higher pressure, followed by treatment of the overhead vapor by one or two membrane steps. However the disclosed production system and process is quite complex and investment intensive, as it requires a vapor compressor, condenser and a liquid feed pump and associated compression, condensation and liquid pressure increasing steps after the first distillation column and before the second distillation column. As a comparison, a "hybrid" distillation process involving two columns connected in both series and parallel is also disclosed. This hybrid process is quite energetically intensive as it requires two distillations, one in each column, as the distillate from the first column is condensed in a condenser and then fed by a pump to the second column. As discussed in US '678, such hybrid processes work well for relatively high concentrations of ethanol; however, for concentrations of ethanol of about 5 wt % or less, they require too much energy input to be efficient and economical. This is a very significant drawback, as the major current commercial interest, especially for second generation biofuels (e.g. ethanol) feed typically obtained from such biomass as lignocellulosic biomass or woody crops, agricultural residues or waste, is for low ethanol concentrations of about 5 wt % or even less.

### SUMMARY OF THE INVENTION

Starting from this state of the art, it is a first object of the invention to provide an improved and robust system for the purification of an organic solvent and secondly an improved process for the purification of an organic solvent using such a system based on a combination of distillation and membrane separation, particularly in terms of increased energy efficiency (reduced operating costs) and reduced complexity and investment.

Further objects of the invention include providing a process for using said system and a use of said system or process in the purification and/or drying of an alcohol, preferably a second generation one, more preferably bioethanol.

Preferably these objectives will be obtained with a system that is simple to build and operate (control) and with a high energy efficiency and availability (i.e. reduced downtime for cleaning operations) and able to operate continuously such that not only the distillation but also the dehydration is continuous.

According to the invention, these objects are achieved by a system for the purification of an organic solvent, preferably an alcohol, comprising in fluid communication: a first distillation column, a second distillation column, a vapor permeation unit suitable for the dehydration of an organic solvent and comprising either zeolite or polymeric pervaporation/vapor permeation membranes,
and wherein the system further comprises: a first heat integration sub-system for exploiting both a sensible heat and optionally a latent heat, and wherein the first heat integration sub-system is embodied for preheating a feed and for cooling a stillage from the first and second columns and optionally for condensing a vapor from a top region of the first distillation column, a second and third heat integration sub-systems for exploiting a latent heat, and wherein the second heat integration sub-system is embodied for condensing a vapor from the second distillation column and vaporizing a liquid in the first distillation column, and wherein the third heat integration sub-system is embodied for heating by means of a retentate vapor EITHER one of the first or second distillation columns OR an optional evaporator unit, wherein an outlet of the optional evaporator unit, when present, is in fluid communication
with an inlet of an optional compressor, and wherein an outlet of the optional compressor, when present, is in fluid communication with an inlet of the vapor permeation unit,

AND wherein there is EITHER a parallel configuration of the system, in which there is a split feeding into inlets of both the first and the second distillation columns and where there is no direct fluid communication between the first and second distillation columns,

OR there is a series configuration of the system, in which there is feeding into only an inlet of the first distillation column and there is a fluid communication between a first outlet of the first distillation column and an inlet of the second distillation column.

Providing such a simplified system based on only two columns reduces the investment cost relative to prior art systems such as those typically disclosed in US2007/0000769 A1. Furthermore, the optimization of the process conditions made possible by the integrated membrane system (vapor permeation unit) in the system of the present invention means that it is not required to have azeotropic compositions coming out of the rectification column before the final drying stage so that the overall energy demand is considerably reduced in the present invention. Molecular sieves, as in US'769 A1, must operate much closer to the aezotropic composition, which requires more reflux in the rectification column and thus the energy demand increases. Molecular sieves require about 25 % recycle of the final product (e.g. dry ethanol) for their semi-continuous regeneration. In contrast, the use of membranes makes possible a continuous removal of the permeate, and it requires no extra energy for regeneration. Furthermore, operating further away from azeotropic conditions with molecular sieves is particularly energy demanding because shorter regeneration cycles become necessary, which thereby increases the amount of recycle (regenerate). In contrast, with membranes there is only a small recycle stream (permeate).

The provision of the first, second and third heat integration sub-systems in the present invention also contribute significantly to the reduction of the energy consumption of the system. For example, in the first heat integration sub-system, it has been found to be beneficial to use the stillage as the heat source for preheating the feed because there is a balance between the heat provided by the stillage and the heat required for preheating the feed. Although there is not an identical flow rate in and out of the system because organic solvent (ethanol) is removed, there is a compensating increase in temperature in the columns. Thus the necessary heat flow rates are nonetheless balanced.

In comparison to the systems disclosed in US 9,138,678 B2, the present invention has the previously-described advantages of providing a system that is simple to build and operate (control) and with a higher energy efficiency and availability (i.e. reduced downtime for cleaning operations because of optimized operating conditions and adapted equipment design for high fouling applications). For example, the system of the present invention lacks the earlier discussed complex compressing, condensing and pumping processes between the two columns, as in US '678 B2. As will be discussed later, the system of the present invention may also have a compressor in some embodiments; however it would be at a different location and for a completely different purpose. In addition, US '678 B2 does not disclose either the first or third heat integration sub-system of the present invention. This is a serious technical disadvantage, as it is noted that about 60 - 75% of the heat required for the evaporation of the distillate is being exploited in the third heat integration sub-system of the present invention.

According to the invention, the second object is achieved by a process for the purification of an organic solvent, preferably an alcohol, using the system of the present invention, in which both a sensible heat and optionally a latent heat are exploited in a first heat integration sub-system and a latent heat is exploited in a second and third heat integration sub-systems, the process further comprising the following sequence of steps of EITHER:
A. wherein there is a parallel configuration of the system:
   - feeding a feed solution comprising an organic solvent to be purified into inlets of both the first and the second distillation columns,
   - concentrating the organic solvent to 85 to 95 wt % and removing a stillage by means of second outlets in the first and second distillation column,
   - condensing the concentrated organic solvent,
   - evaporating the condensed concentrated organic solvent,
   - dehydrating the evaporated concentrated organic solvent to 97 to 99.99 wt % in the vapor permeation unit;
   OR
B. wherein there is a series configuration of the system:
   - feeding a feed solution comprising an organic solvent to be purified into only the inlet of the first distillation column,
   - preconcentrating the organic solvent and removing a stillage in the first distillation column,
   - further concentrating the organic solvent from the first distillation column in the second distillation column by removing a process water and concentrating organic solvent to 85 to 95 wt %,
   - dehydrating the concentrated organic solvent to 97 to 99.99 wt % in the vapor permeation unit.

Said process of using the system of the present invention has the same advantages as those just discussed for the system and will not be repeated here. In a preferred embodiment of the process, the stillage removed from the bottom of either one or both columns is a quasi-alcohol free stillage, typically containing less than 0.1 wt % alcohol.

In a preferred embodiment of the parallel configuration system of the present invention, the system additionally comprises a guard filter unit suitable for the removal of trace acidic species, wherein both the first outlet of the first distillation column and the first outlet of the second distillation column are in fluid communication with an inlet of the guard filter unit and an outlet of the guard filter unit is in fluid communication with an inlet of the vapor permeation unit. In the case of the process of using this system, the process additionally comprises the step of removing trace acidic species from the evaporated concentrated organic solvent in a guard filter unit. The use of a guard filter provides the advantages of increasing the robustness and longevity of the dehydration system (vapor permeation unit and its membranes) by removing undesirable and potentially-harmful trace components such as acidic species, ammonia and acetic acid and their derivatives.

In another preferred embodiment of the system and process of the present invention, the system has the parallel configuration and the split feed into each of the first and second distillation columns is by means of an inlet located in a middle region of each column. This means of split feeding in this configuration of the system provides the advantages of helping to assure that the necessary product qualities are obtained directly in each of the columns and enables the operation and efficiency of the second heat integration sub-system in order to achieve the lowest specific energy or heat demand for the purification of the organic solvent (e.g. ethanol).

In one preferred arrangement of the parallel configuration, the third heat integration sub-system is embodied for heating the evaporator unit by means of a retentate vapor. This arrangement provides the advantages of enabling about 60 - 75% of the heat required for the evaporation of the distillate to be obtained via the third heat integration sub-system of the present invention. Surprisingly it has been found that if a compressor is additionally present, there is little or no need for extra steam for the evaporation.

In a preferred embodiment of the parallel configuration, the first and second distillation column each comprises in vertical sequence from top to bottom: a rectification section, a stripping section, optionally comprising antifouling trays, and an evaporator or a live steam injector. This embodiment provides the advantages of enabling an optimum performance and basis for the second and third heat integration sub-systems and reducing the specific energy or heat demand for the purification of the organic solvent (e.g. ethanol). Low to medium pressure steam may be utilized for heating in this embodiment, which then increases the versatility in relation to the utility requirements.

In another preferred embodiment of the system having a parallel configuration, the first and second distillation column each have a second outlet in a middle region of each column embodied for the discharge of a fusel oil. This embodiment has the advantage of preventing fusel oil accumulation, which would cause negative effects on the product quality and may also cause damage to the system's equipment if the fusel oil accumulates too long inside the system. It is also important to note that besides fusel oil, the discharge contains water and valuable organic solvent, preferable ethanol. Optionally the fusel oil may be separated from the organic solvent (e.g. ethanol) which is recycled thus increasing the organic solvent (e.g. ethanol) yield of the plant to >99 wt %. For example, if there is a 600 ppm ethanol loss in the stillages and no fusel oil recovery, about 1.4 wt % of the total ethanol in the feed is lost. If however there is a fusel oil recovery, the recovery rate will be at least 99 wt %. In the case of 1'000 ppm EtOH in the stillages, the loss will be closer to 2 wt %. Thus it is beneficial to limit the loss of ethanol in the stillages.

In still yet another preferred embodiment of the parallel configuration, the system additionally comprises one or two degassers, wherein an outlet of the one or two degassers are each in fluid communication with an inlet of the first and second distillation column. Thus preferably there may be one degasser present and in fluid communication with the inlets of both columns or there may be two degassers present, each being in fluid communication with an inlet of each column. This provision of a degasser has the advantage of depleting small quantities of unwanted low boiling components such as methanol and/or removing traces of inerts such as CO₂ from the feed stream. These unwanted components generally have negative effects on the product quality, such as increasing the acidity above the limits defined in the regulatory norms.

In yet another preferred embodiment of the parallel configuration, the system additionally comprises a first and second centrifuges, wherein a second outlet of each of the first and second distillation column are each in fluid communication with an inlet of one of the centrifuges. Likewise the process of using this system includes the step of treating a stillage removed from the first and second distillation column in a first and a second centrifuge for removing a suspended biomass. Providing centrifuges has the advantage of removing suspended biomass as early as possible after the organic solvent (e.g. ethanol) is removed, which then increases the effectiveness of the centrifuge, eases the plant operation, and increases plant availability. Depending on the nature of the raw material used as feed to the upstream plant, the resulting stillage may contain valuable products, which may then be concentrated and sold. Alternatively, if technically feasible, the stillage may be partially recycled to the upstream process in order to reduce the operational cost, such as waste water treatment cost.

In one preferred embodiment of the parallel or series configuration, the second heat integration sub-system is embodied for heating the first distillation column by means of a retentate vapor. This embodiment also has the advantage of reducing the specific energy or heat demand for the purification of ethanol. It is noted that generally the retentate is not sufficient for providing the entire heating of the column, and an auxiliary heating source is typically required. For series arrangement this embodiment is particularly helpful because additional heat in the bottom of the first column is required.

In one preferred embodiment of the series configuration system, the first outlet of the second distillation column is in fluid communication with an inlet of the guard filter unit. Likewise the process of using this system additionally comprises the step of removing trace acidic species from the vapor-phase concentrated organic solvent obtained from the top region of the second distillation column in a guard filter unit. The advantages of providing a guard filter have been discussed earlier for the parallel configuration.

In another preferred embodiment when the system has a series configuration, the system additionally comprises a condenser, wherein the inlet for the feed into the first distillation column is in an upper region of the column and wherein the first outlet of the first column is in a top region of the column and in fluid communication with an inlet of the condenser, wherein an outlet of the condenser is in fluid communication with the first inlet into a middle region of the second column. The provision of this means of feeding into the second column advantageously enables the two columns to operate under differing conditions and that vapors are readily produced in the second column which may then be sent to the vapor permeation unit.

In yet another preferred embodiment when the system has a series configuration, the first distillation column lacks a rectification section and comprises in vertical sequence from top to bottom: a stripping section, an evaporator and/or a live steam injector, and the second distillation column comprises in vertical sequence from top to bottom: a rectification section, a stripping section, and an evaporator and/or a live steam injector. This configuration of the columns has the advantage of a low specific energy or heat demand for the purification of organic solvent (e.g. ethanol) and eliminates the need for providing an evaporator before the vapor permeation unit. Additionally the process water from the bottom of the second column can beneficially be recycled to the upstream units, such as a fermenter.

In yet another preferred embodiment of the series configuration of the system, a first outlet for a vapor in a top region of the second distillation column is in direct fluid communication with both an inlet of the guard filter unit and the evaporator within the first distillation column. This specific configuration has the advantage of increasing the robustness and longevity of the dehydration system (vapor permeation unit and its membranes) by removing undesirable and potentially-harmful trace components such as acidic species, particularly acetic acid, and ammonia and their derivatives.

In still yet another preferred embodiment of the system having a series configuration, the second distillation column has a second outlet in a middle region embodied for the discharge of a fusel oil. The advantages of this discharge on product quality and in preventing equipment damage have been discussed earlier.

In still yet another preferred embodiment of the series configuration, the system additionally comprises a degasser, wherein an outlet of the degasser is in fluid communication with an inlet of the first distillation column. The advantages in providing a degasser in terms of product quality have been described earlier.

In yet another preferred embodiment of the series configuration, the system additionally comprises a first centrifuge, wherein a second outlet of the first distillation column is in fluid communication with an inlet of the first centrifuge. Likewise in the process of using this system a stillage removed from the first distillation column is treated in the first centrifuge for removing a suspended biomass. The advantages of a centrifuge in an early removal of suspended biomass after organic solvent (e.g. ethanol) removal have been discussed earlier.

In a preferred embodiment of the process of the present invention, the first column is operated at a temperature of from about 70 to about 90°C and the second column is operated at a temperature of from about 90 to about 120°C in the case that the system has a parallel configuration and at a temperature of from about 120 to about 160 °C in the case of the series configuration. The operation of the columns in these temperature ranges has the advantage that the important contribution of the first and second heat integration subsystems to reducing the specific energy or heat demand for the purification of the organic solvent (e.g. ethanol) is being achieved. Also, the favorable low operating temperatures when biomass is present reduces the fouling tendency, which is especially important for certain second generation raw materials.

One skilled in the art will understand that the combination of the subject matters of the various claims and embodiments of the invention is possible without limitation in the invention to the extent that such combinations are technically feasible. In this combination, the subject matter of any one claim may be combined with the subject matter of one or more of the other claims. In this combination of subject matters, the subject matter of any one process claim may be combined with the subject matter of one or more other process claims or the subject matter of one or more system claims or the subject matter of a mixture of one or more process claims and system claims. By analogy, the subject matter of any one system claim may be combined with the subject matter of one or more other system claims or the subject matter of one or more process claims or the subject matter of a mixture of one or more process claims and system claims. By way of example, the subject matter of any one claim may be combined with the subject matters of any number of the other claims without limitation to the extent that such combinations are technically feasible.

One skilled in the art will understand that the combination of the subject matters of the various embodiments of the invention is likewise possible without limitation in the invention. For example, the subject matter of one of the above-mentioned preferred system embodiments may be combined with the subject matter of one or more of the other above-mentioned preferred process embodiments or vice versa without limitation so long as technically feasible.

### BRIEF DESCRIPTION OF THE DRAWINGS

The invention will be explained in more detail hereinafter with reference to various embodiments of the invention as well as to the drawings. The schematic drawings show:
- Fig. 1: shows a schematic view of one embodiment of a parallel configuration of the system of the present invention having a third heat integration sub-system embodied for heating an evaporator unit.
- Fig. 2: shows a schematic view of a preferred embodiment of a parallel configuration of the system of the present invention having a third heat integration sub-system embodied for heating the second column.
- Fig. 3: shows a schematic view of a preferred embodiment of a series configuration of the system of the present invention.

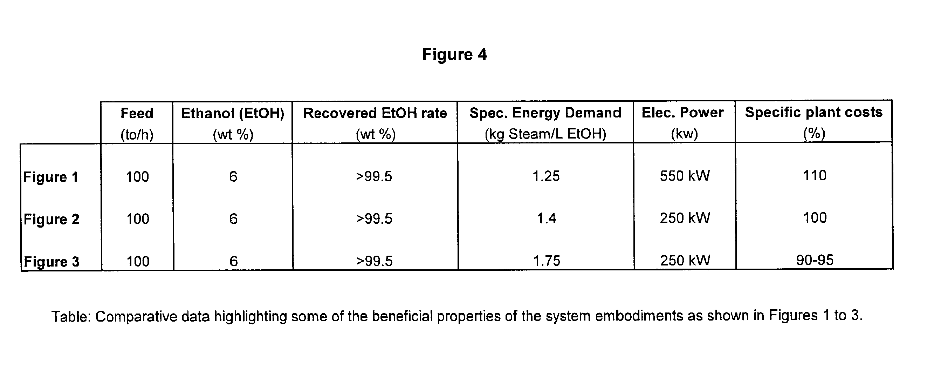

- Fig. 4: shows a comparative table of some of the beneficial properties of the system embodiments shown in Figs. 1 to 3

### DETAILED DESCRIPTION OF THE INVENTION

### DEFINITIONS

As used in the specification and claims of this application, the following definitions, should be applied:
"a", "an", and "the" as an antecedent may refer to either the singular or plural unless the context indicates otherwise.

"Organic solvent" in the present application refers to common organic solvents as known in the art and thus including alcohols, ketones, aldehydes, and esters; such as ethanol, particularly bioethanol produced from natural sources (C2); butanol (C4); acetone (C3); and ABE.

For series arrangement, the first column is often referred to in the art as a "beer column" or "mash column" and the second column is referred to as a "rectifying column". In contrast for the parallel arrangement, both the first and second column are the same type of column, typically comprising an upper region (rectifying section) and a middle region (stripping section), but operated under different operating conditions.

Concerning the location of particular regions within a column in the present application, the "top region" is above the "upper region" (typically the rectifying section), which is above the "middle region" (typically the stripping section) of the columns. The "bottom region" is located below the "middle region".

The term "guard filter" in the present application refers to an unit up-front of the vapor permeation unit (VPU, e.g. membrane module) having the function of eliminating unwanted by-products such as trace concentrations of acidic species or ammonia. The elimination of these species from the stream fed to the VPU acts to beneficially increase the robustness and lifetime of the pervaporation and/or vapor permeation membranes in the application.

The term "fusel oil" in the present application refers to a mixture of several C3-C5 alcohols (often amyl alcohol) that are produced as a by-product of alcoholic fermentation. The components of the mixture have similar physical behavior, e.g. as middle boiling components, and they have an oily consistency.

The term "membrane" in the present application refers to dense membranes which act as a selective barrier between two phases namely, a liquid-phase feed and a vapor-phase permeate. The membrane allows the desired component(s) of the liquid feed to transfer through it by vaporization. Separation of components (e.g. water and ethanol) is based on a difference in transport rate of individual components through the membrane. The membranes may be either polymeric- or zeolite-based in composition. The feed to the membrane may either be in the liquid phase (pervaporation process) or in the vapor phase (vapor permeation process).

Numerical values in the present application relate to average values. Furthermore, unless indicated to the contrary, the numerical values should be understood to include numerical values which are the same when reduced to the same number of significant figures and numerical values that differ from the stated value by less than the experimental error of the conventional measurement technique of the type described in the present application to determine the value.

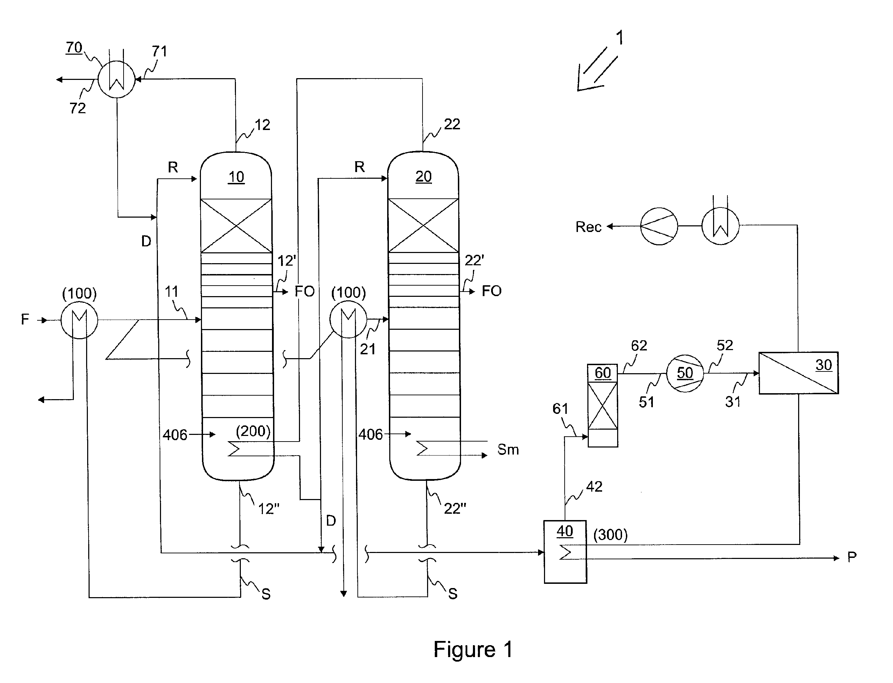

FIG. 1 shows a schematic view of one embodiment of a parallel configuration of the system of the present invention having a third heat integration sub-system embodied for heating an evaporator unit, which as a whole is labeled with reference number 1. The system 1 is not specifically limited as to form, shape, construction or composition unless specifically indicated otherwise. Any suitable material that can be fabricated can be made into system 1. For reasons of economy, such systems 1 are often made from stainless steel or another material indicated for the specific application. System and column internal components are generally made from metals depending upon the process requirements. In one embodiment the system 1 and its components are constructed of metals. Suitable metals include carbon steel, stainless steel, nickel alloys, copper alloys, titanium and zirconium.

Columns and their construction and operation are well known in the art, for example, as disclosed in chapter 5 of Handbook of Separation Process Technology by Ronald W. Rousseau, published by John Wiley in 1987 (ISBN 0-471-89558-X) and Fundamentals and modeling of separation processes: absorption, distillation, evaporation and extraction, by C.D. Holland, published in 1975 by Prentice-Hall (ISBN 0-13-344390-6). Unless indicated otherwise, conventional construction materials and means, as well as components and auxiliaries, may be used for the system 1, and the system 1 may be operated in a separation process in a conventional manner as known in the art. For example, these cited reference handbooks and textbooks disclose a variety of conventional means for evaporating, heat exchanging and condensing for use in separation systems. It is noted that in many embodiments of the invention, such as those shown in the Figures, the system will have only two distillation columns, namely the first and second distillation columns 10, 20.

Suitable feed solutions in the present invention include mixtures of one or more organic compounds in the mixture to be separated. A typical example of a feed solution that may be successfully treated by means of the present invention is ABE, an acetone-butanol-ethanol mixture produced, for example, by fermentation, and used as a source of bio-butanol and other valuable chemical feedstocks. In such a case, an aqueous mixture of organic solvents is separated from the broth, and the organic solvent mixture is then dehydrated by the membranes in the VPU.

The feed solution in the present invention may contain additional components besides organic solvents and water, such as inorganic salts, fermentation debris, etc. The source of the feed solution is not specifically limited, and it may be subjected to pre-treatment, such as filtration, to remove contaminants before it enters the system 1. Such contaminants may also be removed by side draws of the first and/or second column 10 and 20. Such pretreatment and side processes do not normally affect the processes of the invention.

Representative sources of the feed solution include processes that manufacture organic solvents and processes that use organic solvents. Feed solutions that are particularly well suited to treatment by the system 1 and process of the present invention are those from the manufacture of low molecular weight and thus low boiling alcohols, ketones, aldehydes, organic acids and esters by means of industrial chemical synthesis or fermentation. Such manufacturing processes may include chemical syntheses from petrochemical feedstocks, such as olefins; fermentation of sugar-containing feedstocks; hydrolysis/saccharification/fermentation of cellulosic and lignocellulosic feedstocks; and the conversion of carbon-containing materials to a chemical feedstock, followed by chemical or biochemical production of the desired solvent from the feedstock or its intermediate or derivative.

The system 1 of Fig. 1 has the parallel configuration having a third heat integration sub-system embodied for heating an evaporator unit. This embodiment of the third heat integration sub-system is particularly useful if there are steam pressure limitations on the site. This third heat integration sub-system in Fig. 1 is particularly advantageous as it requires essentially no steam for the dehydration step.

Some possible types of compressors 50 suitable for use in the parallel configuration of the system 1 include centrifugal or radial compressors.

Some evaporator 40 types suitable for the parallel configuration include natural or forced thermosiphon evaporators executed as kettle type, vertical or horizontal shell and tube or plate heat exchangers.

In the parallel configuration, the first and second columns 10 and 20 will typically operate at pressures that enable the proper operation of the second and third heat integration sub-systems 200 and 300 while also not inducing excessive fouling of the biomass. This is particularly important in the parallel configuration as typically both columns then contain biomass. It is noted that the first and second columns 10 and 20 typically will partially contain packing in order to reduce the pressure drop so as to facilitate their heat integration sub-systems.

Of great importance to an optimum process when using the parallel arrangement is the feed split percentage between the two columns 10 and 20. In order to make possible a maximum recovery of energy by the heat integration sub system 200, a feed split percentage to each column of between about 40 to about 60 %, most preferable about 50 % to each column is used.

The parallel configuration system 1 shown in Fig. 1 has a higher electrical consumption compared to other embodiments of the invention as electricity is required for driving the compressor of the third heat integration sub-system 300.

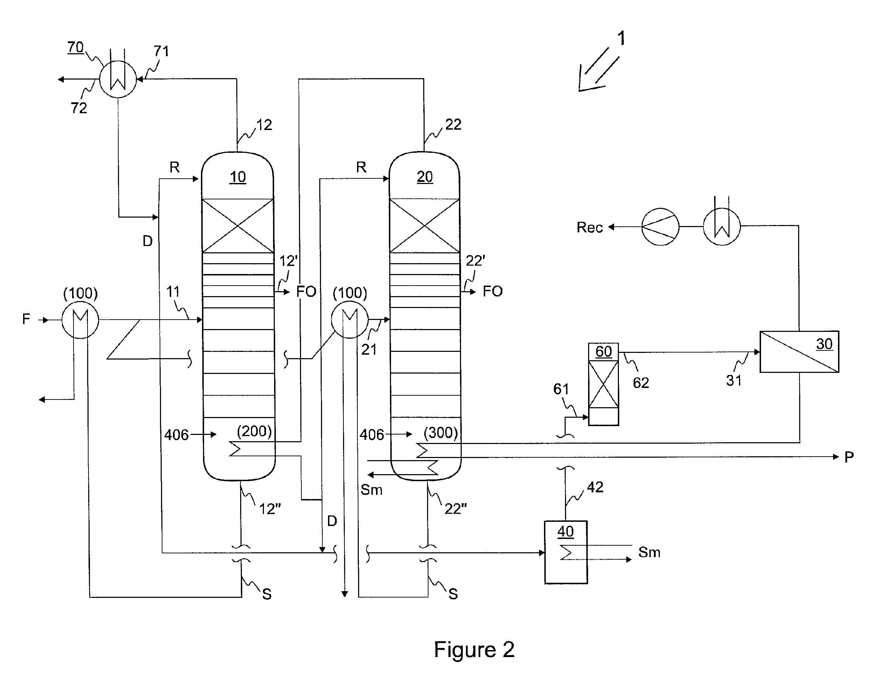

Fig. 2 shows a schematic view of one preferred embodiment of a parallel configuration of the system 1 of the present invention having a third heat integration sub-system 300 embodied for heating the second column 20. This third heat integration sub-system 300 has advantages over the one shown in Fig. 1 in that it is simpler and less expensive due to its lack of a compressor. However such a third heat integration sub-system 300 requires generally a medium pressure steam, such as about 5 - 6 bar; whereas the one in Fig. 1 requires only about 3 - 4 bar. Therefore if steam supply pressure is no limitation, typically the embodiment shown in Fig. 2 is often preferred over that of Fig. 1 due to cost and maintenance reasons.

It is noted that parallel configurations of the system 1, particularly those as in Fig. 2, will generally be preferred versus series configurations, as the specific energy demands will often be lower and the costs of the system 1 are not significantly higher.

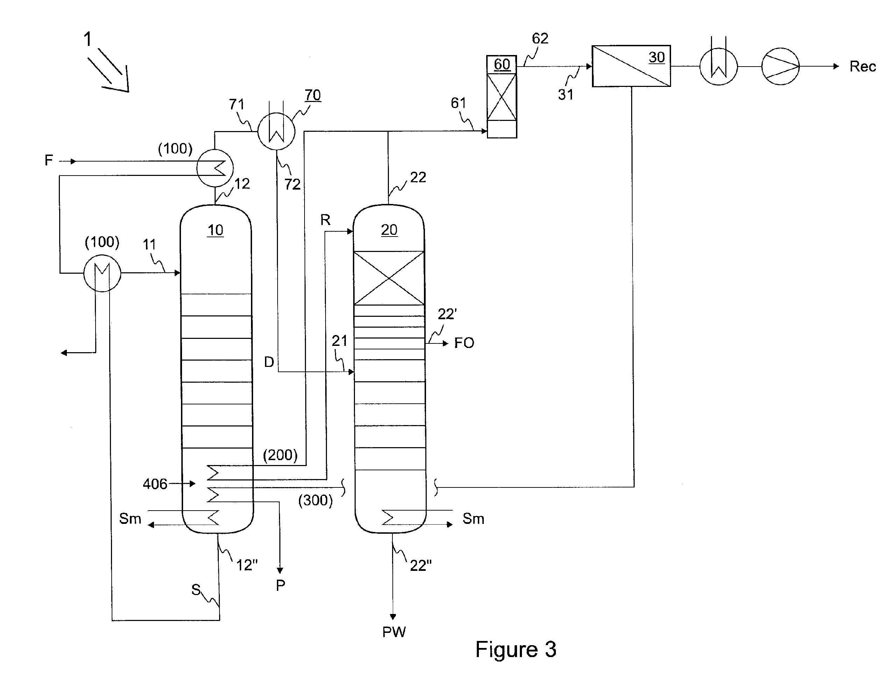

Fig. 3 shows a schematic view of a preferred embodiment of a series configuration of the system 1 of the present invention. This embodiment has the advantage of reduced cost and complexity in that it lacks complex equipment such as a compressor. In addition, the second column 20 operates at a higher temperature and pressure so that it directly generates vapors for feeding to the vapor permeation unit 30 (thus requiring less equipment). However the cost reduction versus the parallel configuration is only quite modest, e.g. typically about 5 to perhaps 10 %. On the other hand, a disadvantage is that the specific energy demand increases by about 25 % for this series embodiment of the system 1 versus that of the parallel embodiment shown in Fig. 2.

The higher operating temperature in the second column 20 allows for a greater temperature difference in the first and second heat integration sub-systems 100 and 200, and thus the sub-systems 100 and 200 may be constructed smaller, which will also reduce costs. Due to the typically slightly higher operating temperature in the first column 10 in the series arrangement, there are less stringent cooling water requirements for the vapors leaving the first column 10.

Important to note is that the biomass wetted part of the system 1 is generally limited to the first column 10. Therefore fouling does not occur in the second column 20. However it should be noted that the series configuration typically has the disadvantage of requiring higher steam pressures of about 8 to 10 bar.

The guard filter unit 60 up-front of the VPU 30 (e.g. membrane modules) suitable for either the parallel or series configuration of the system 1 has the function of eliminating undesired by-products present in trace concentrations such as acidic species or ammonia. Thus the guard filter typically comprises materials suitable for binding and removing trace acids from the stream fed to the VPU 30. Alternatively the acids may be converted to neutral species. The guard filter materials will thus often have ion-exchange properties and may be inorganic or polymeric in nature. The elimination of such trace acidic species is of considerable importance when changing feed concentrations or operating conditions, particularly during start-up and shut-down, which are generally the most stressful periods in dealing with complex systems and their operation. The guard filter unit 60 thus brings important advantages in considerably increasing the lifetime of the pervaporation and/or vapor permeation membranes and the robustness and minimizing plant maintenance efforts and costs.

Integrated pervaporation/vapor permeation membranes suitable for use in the VPU 30 in either the parallel or series configuration fulfill the important function of minimizing the overall energy demand of the downstream section. The regeneration of the membranes may favorably be carried out continuously and requires no additional energy. The aqueous permeate typically contains only small amounts of solvent in the present invention. In contrast, the semi-continuous regeneration of molecular sieves in conventional systems requires much higher regeneration streams, usually involving up to about 25 % of the total dry ethanol produced. It is noted that the driving force of the membranes is increased at higher water concentrations. As a consequence the present invention then favors higher water concentration in the feed to the dehydration section which considerably reduces the reflux ratio in the preceding first and second columns 10 and 20. It is noted that normally a membrane type will be selected that is not affected by lower concentrations of acetaldehyde.

Applications containing biomass prone to fouling generally requires the careful selection of column internals. Plugging of column internals reduces plant capacity and efficiency, requires frequent shutdowns, causes loss of operation time and increases maintenance costs. Thus careful selection of operating and hydraulic conditions as well as suitable types of internals, preferable trays are of the essence. V-grid tray type without any moving parts and special designed push valves to prevent stagnations zones are examples of suitable trays for maintaining both a high tray efficiency and increasing plant availability. It is noted that Sulzer Chemtech's V-grid antifouling trays are widely used, especially in so-called beer or mash columns.

Although not shown in the schematic figures for simplicity, one skilled in the art will understand that other conventional column and separation device internals may be used without limitation in the invention, such as feed devices like feed pipes and/or sumps, bed limiters, support plates and grids, dispersers, disperser/support plates, continuous phase distributors, packing support and hold-down plates, mist eliminators, collectors, entrainment separators, and retainers/redistributors. Suitable internals are disclosed for example in the technical brochure "Internals for Packed Columns" from Sulzer Chemtech as publication 22.51.06.40 - XII.09 - 50.

Auxiliaries for the system 1 are conventional and well-known in the art and include electrical supplies, coolant and heating fluid supplies and distributions, level controllers, pumps, valves, pipes and lines, reservoirs, drums, tanks, and sensors for measuring such parameters as flow, temperatures and levels. The system 1 and the separation process will be conveniently controlled by means of a computer interface equipped with appropriate sensors.

Distillation and separation processes are well known in the art, for example, as disclosed in the earlier cited text- and reference books. Unless indicated otherwise, conventional distillation and pervaporation processes and their various feed streams and operating parameters and conditions may be used in the separation processes according to the invention and making use of the system 1.

This separation process of the invention has the benefit of making possible the production of organic solvents at modest capital costs with a very low greenhouse gas (GHG) footprint for different biofuel production systems, especially suited for diluted feedstock arising from second generation (2G) based raw materials. For diluted feed solutions, the specific energy demand is one key performance indicator for the downstream purification of a solvent that can become easily too high and thus uneconomical. In a worst case, the energy demand for separating an organic solvent (e.g. ethanol) from a dilute aqueous solution is higher than the heating value obtained when the solvent (e.g. ethanol) is blended as biofuel in gasoline.

Typical concentrations of organic solvent in the feed solution and the various streams of the system 1 and process of the invention depend strongly on the type of solvent, the type of raw material and the selected process. As examples, ethanol and butanol are mentioned.

The concentration of ethanol in the feed solution is typically between about 3 to 15 wt % depending on the raw material source, for example, a first or second generation one. In the case of butanol it will generally be between about 1 to about 3 wt % depending on the specific raw material and fermentation process used in its preparation.

The process of pre-concentrating the organic solvent (e.g. ethanol) in the series configuration of the system 1 is dependent on the concentration in the feed. Generally the concentration varies between about 25 to about 60 wt % after pre-concentration in the distillate of the first column 10.

The typical composition of the bottom stream (stillage or process water) contains less than about 1,000 ppm of organic solvent (e.g. ethanol). A higher solvent concentration reduces the recovery yield, increases the waste water treatment cost or even prevents the recovery of valuable components of the stillage for use as a sellable product, e.g. as cattle feed.

In cases where the organic solvent is ethanol, preferred concentrations of other selected streams for the production of ethanol are as follows. Fusel oil discharge having a composition of about 1/3 fusel oil, about 1/3 ethanol, and about 1/3 water. The composition of the vent gas will depend on the method of vent gas treatment, e.g. if treated by an absorber the vent gas may contain only trace amounts of ethanol, for example, as low as less than 1,000 ppm ethanol. The composition of the feed to the dehydration step in the VPU will generally contain about 85 to about 95 wt % ethanol.

### EXAMPLES

The following examples are set forth to provide those of ordinary skill in the art with a detailed description of how the system 1 adapted for the purification of an organic solvent therein, processes, and uses claimed herein are evaluated, and they are not intended to limit the scope of what the inventors regard as their invention.

In these examples, embodiments of the system 1 similar to the ones shown in Figs. 1 to 3 are compared as to their beneficial advantages for the base case of a feed flow rate of 100 ton/h and an ethanol concentration in the feed solution of 6 wt %. The three systems 1 are operated so as to produce a dry ethanol product of 99.5 wt % and a stillage containing less than 1'000 ppm ethanol.

For comparison purposes, it is noted that conventional first generation bioethanol plants known in the art have the specific energy demand of at least about 1.2 to about 2 kg Steam/L ethanol produced or more when the ethanol concentration in the feed solution is about 11 to about 15 wt %. For the parallel configuration systems 1 shown in Figs. 1 and 2, the specific energy demand is significantly less than 1 kg Steam/ L ethanol produced for the case of about 11 to about 15 wt % ethanol in the feed solution. One skilled in the art will understand that a detailed comparison of the steam consumption and specific energy demand will require a consideration of such process parameter aspects as the particular feed solution composition as well as the exact design of the plant (e.g. the number of columns).

While various embodiments have been set forth for the purpose of illustration, the foregoing descriptions should not be deemed to be a limitation on the scope herein. Accordingly, various modifications, adaptations, and alternatives can occur to one skilled in the art without departing from the spirit and scope herein.

### Reference Symbols

D distillate
F feed solution
FO fusel oil
P product
PW process water
R reflux
Rec recycle
S stillage
Sm steam
1 system
10 first distillation column
11 inlet of the first distillation column 10
12 first outlet of the first distillation column 10
12' second outlet of the first distillation column 10
12" third outlet of the first distillation column 10
20 second distillation column
21 inlet of the second distillation column 20
22 first outlet of the second distillation column 20
22' second outlet of the second distillation column 20
22" third outlet of the second distillation column 20
30 vapor permeation unit
31 inlet of the vapor permeation unit 30
40 optional evaporator unit
42 outlet of the optional evaporator unit 40
50 optional compressor
51 inlet of the optional compressor
52 outlet of the optional compressor
60 guard filter unit
61 inlet of the guard filter unit 60
62 outlet of the guard filter unit 60
70 condenser of the first distillation column
71 inlet of the condenser
72 outlet of the condenser
100 first heat integration sub-system
200 second heat integration sub-system
300 third heat integration sub-system
400 rectification section
402 stripping section
404 evaporator
406 live steam injector
500 degasser
600 first centrifuge
601 inlet of first centrifuge
700 second centrifuge
701 inlet of second centrifuge

## Claims

1. A system (1) for the purification of an organic solvent, preferably an alcohol, comprising in fluid communication:
- A first distillation column (10),
- A second distillation column (20),
- A vapor permeation unit (30) suitable for the dehydration of an organic solvent and comprising either zeolite or polymeric pervaporation/vapor permeation membranes,
wherein the system (1) further comprises:
- a first heat integration sub-system (100) for exploiting both a sensible heat and optionally a latent heat, and wherein the first heat integration sub-system (100) is embodied for preheating a feed (2) and for cooling a stillage (3) from the first and second columns (10, 20) and optionally for condensing a vapor from a top region of the first distillation column (10),
- a second and third heat integration sub-systems (200, 300) for exploiting a latent heat, and wherein the second heat integration sub-system (200) is embodied for condensing a vapor from the second distillation column (20) and vaporizing a liquid in the first distillation column (10),
and wherein the third heat integration sub-system (300) is embodied for heating by means of a retentate vapor EITHER one of the first or second distillation columns (10, 20) OR an optional evaporator unit (40), wherein an outlet (42) of the optional evaporator unit (40), when present, is in fluid communication with an inlet (51) of an optional compressor (50), and wherein an outlet (52) of the optional compressor (50), when present, is in fluid communication with an inlet (31) of the vapor permeation unit (30),
AND wherein there is EITHER a parallel configuration of the system (1), in which there is a split feeding into inlets (11, 21) of both the first and the second distillation columns (10, 20) and where there is no direct fluid communication between the first and second distillation columns (10, 20),
OR there is a series configuration of the system (1), in which there is feeding into only an inlet (11) of the first distillation column (10) and there is a fluid communication between a first outlet (12) of the first distillation column (10) and an inlet (21) of the second distillation column (20).

2. The system (1) of claim 1, wherein the system (1) additionally comprises a guard filter unit (60) suitable for the removal of trace acidic species, wherein an outlet (62) of the guard filter unit (60) is in fluid communication with an inlet (31) of the vapor permeation unit (30), and EITHER wherein in the case of the parallel configuration of the system, both the first outlet (12) of the first distillation column and the first outlet (22) of the second distillation column (20) are in fluid communication with an inlet (61) of the guard filter unit (60),
OR wherein in the case of the series configuration of the system, the first outlet (22) of the second distillation column (20) is in fluid communication with an inlet (61) of the guard filter unit (60).

3. The system (1) of either one of claims 1 or 2, wherein the system (1) has the parallel configuration and wherein the split feed into each of the first and second distillation columns (10, 20) is by means of an inlet (11,21) located in a middle region of each column (10, 20).

4. The system (1) of claim 3, wherein the third heat integration sub-system (300) is embodied for heating the evaporator unit (40) by means of a retentate vapor.

5. The system (1) of any one of claims 1 to 4, wherein the second heat integration sub-system (200) is embodied for heating the first distillation column (10) by means of a retentate vapor.

6. The system (1) of any one of claims 3 to 5, wherein the first and second distillation column (10, 20) each comprises in vertical sequence from top to bottom:
- a rectification section (400),
- a stripping section (402), optionally comprising antifouling trays,
- and an evaporator (404) or a live steam injector (406).

7. The system (1) of any one of claims 1 to 3, wherein the system has a series configuration and additionally comprises a condenser (70), wherein the inlet (11) for the feed into the first distillation column (10) is in an upper region of the column and wherein the first outlet (12) of the first column is in a top region of the column (10) and in fluid communication with an inlet (71) of the condenser (70), wherein an outlet (72) of the condenser (70) is in fluid communication with the first inlet (21) into a middle region of the second column (20).

8. The system (1) of claim 7, wherein the first distillation column (10) lacks a rectification section (400) and comprises in vertical sequence from top to bottom:
- a stripping section (402),
- an evaporator (404) and/or a live steam injector (406),
and wherein the second distillation column (20) comprises in vertical sequence from top to bottom:
- a rectification section (400),
- a stripping section (402),
and an evaporator (404) and/or a live steam injector (406).

9. The system (1) of either claim 7 or 8 dependent on claim 1 or 2, wherein a first outlet (22) for a vapor in a top region of the second distillation column (20) is in direct fluid communication with both an inlet (61) of the guard filter unit and the evaporator (404) within the first distillation column (10).

10. The system (1) of any one of claims 1 to 9, wherein EITHER the system has a parallel configuration and the first and second distillation column (10, 20) each have a second outlet (12', 22') in a middle region of each column (10, 20) embodied for the discharge of a fusel oil,
OR
the system (1) has a series configuration and the second distillation column (20) has a second outlet (22') in a middle region embodied for the discharge of a fusel oil.

11. The system (1) of any one of claims 1 to 9, wherein either:
- the system (1) has a parallel configuration and additionally comprises one or two degassers (500), wherein an outlet (502) of the one or two degassers (500) are each in fluid communication with an inlet (11, 21) of the first and second distillation column (10, 20), OR
- the system (1) has a series configuration and additionally comprises a degasser (500), wherein an outlet (502) of the degasser (500) is in fluid communication with an (11) inlet of the first distillation column (10).

12. The system (1) of any one of claims 1 to 11, wherein either:
- the system (1) has a parallel configuration and additionally comprises a first and second centrifuges (600, 700), wherein a second outlet (12', 22') of each of the first and second distillation column (10, 20) are each in fluid communication with an inlet (601, 701) of one of the centrifuges (600, 700),
OR
- the system has a series configuration and additionally comprises a first centrifuge (600), wherein a second outlet (12') of the first distillation column (10) is in fluid communication with an inlet (601) of the first centrifuge (600).

13. A process for the purification of an organic solvent, preferably an alcohol, using the system (1) of any one of claims 1 to 12, in which both a sensible heat and a latent heat are exploited in a first heat integration sub-system (100) and a latent heat is exploited in a second and third heat integration sub-systems (200, 300), the process further comprising the following sequence of steps of either:
A. wherein there is a parallel configuration of the system:
- feeding a feed solution comprising an organic solvent to be purified into inlets (11,21) of both the first and the second distillation columns (10, 20),
- concentrating the organic solvent to 85 to 95 wt % and removing a stillage by means of a second outlet (12', 22') in the first and second distillation column (10, 20),
- condensing the concentrated organic solvent,
- evaporating the condensed concentrated organic solvent,
- dehydrating the evaporated concentrated organic solvent to 97 to 99.99 wt % in the vapor permeation unit (30);
OR
B. wherein there is a series configuration of the system (1):
- feeding a feed solution comprising an organic solvent to be purified into only the inlet (11) of the first distillation column (10),
- preconcentrating the organic solvent and removing a stillage in the first distillation column (10),
- further concentrating the organic solvent from the first distillation column (10) in the second distillation column (20) by removing a process water and concentrating organic solvent to 85 to 95 wt %,
- dehydrating the concentrated organic solvent to 97 to 99.99 wt % in the vapor permeation unit (30).

14. The process of claim 13, wherein the system has a parallel configuration and the process additionally comprises the step of removing trace acidic species from the evaporated concentrated organic solvent in a guard filter unit (60), OR
wherein the system has a series configuration and the process additionally comprises the step of removing trace acidic species from the vapor-phase concentrated organic solvent obtained from the top region of the second distillation column (20) in a guard filter unit (60).

15. The process of either one of claim 13 or 14, wherein a stillage removed from a first and/or second distillation column (10, 20) is treated in a first centrifuge (600) for removing a suspended biomass.

16. The process of any one of claims 13 to 15, wherein the first column (10) is operated at a temperature of from about 70 to about 90 °C and the second column (20) is operated at a temperature of from about 90 to about 120 °C when the system (1) has a parallel configuration and at a temperature of from about 120 to about 160 °C when the system (1) has a series configuration.
